# EUROPÄISCHE PATENTSCHRIFT

(11) **EP 1 247 514 B1**
(45) Veröffentlichungstag und Bekanntmachung des Hinweises auf die Patenterteilung: **06.06.2007**
(21) Anmeldenummer: 02007459.7
(22) Anmeldetag: 30.03.2002
(51) Int. Cl.: A61K 8/39, A61Q 1/04

(54) **Flüssigkeit zur Lippenpflege und Lippenfärbung**
Lip care and color liquid
Liquide pour le soin et le maquillage des lèvres

(30) Priorität: 03.04.2001 DE 10116497
(43) Veröffentlichungstag der Anmeldung: 09.10.2002
(73) Patentinhaber: Faber-Castell AG, 90547 Stein (DE)
(72) Erfinder: Frank, Tatiana, 90522 Oberasbach (DE); Lugert, Gerhard, Dr., 90431 Nürnberg (DE); Schwarz, Wolfgang, 90522 Ansbach (DE)
(74) Vertreter: Mörtel & Höfner

(56) Entgegenhaltungen:
- EP-A- 0 336 000
- EP-A- 1 216 686
- EP-A- 1 216 695
- WO-A-01/82877
- WO-A-01/91709
- WO-A-94/26234
- DE-A- 19 642 090
- DE-A- 19 832 889
- US-A- 5 603 926
- US-A- 5 858 340

## Beschreibung

Die Erfindung betrifft eine Flüssigkeit zur Lippenpflege oder Lippenfärbung. Herkömmliche Lippenstifte weisen eine Mine fett- oder wachsartiger Konsistenz auf. Aus US 5,756,014 ist ein Lippenstift bekannt, der Polyoxyethylen-glycerin-monostearat oder Polyoxyethylen-glycerin-monooleat enthält. Um eine ausreichende Färbung zu erreichen, muss eine relativ große Menge auf die Haut aufgebracht werden. Nachteilig dabei ist, dass der Auftrag leicht verwischt und auch leicht wieder abgeht. Ausserdem ist mit den relativ dicken Lippenstiftminen ein liniengenauer Auftrag nicht ganz einfach zu erreichen. Leichter lässt sich dies mit kapillaren Auftragsgeräten, die flüssige Formulierungen benutzen, bewerkstelligen. Eine in US 2,548,970 beschriebene Zusammensetzung für die Lippenfärbung enthält neben einem flüchtigen Lösungsmittel, wie etwa Ethanol oder Isopropylalkohol Benzoeharz, Cetylalkohol und Lecithin. Formulierungen mit Filmbildnern sind aus EP 853 940 und US 4,795,631 bekannt. In DE 196 279 31 ist ein Lippenfärbemittel mit einer Mischung aus Wasser und Isopropylalkohol als Lösungsmittel beschrieben.

Aufgabe der Erfindung ist es, eine Flüssigkeit zur Lippenpflege oder Lippenfärbung mit einer alternativen Zusammensetzung anzugeben, die sich mit einem kapillaren Auftragsgerät auftragen lässt.

Diese Aufgabe wird gemäß Anspruch 1 durch eine Flüssigkeit gelöst, die Wasser und wenigstens ein Feuchthaltemittel als Hauptbestandteile sowie wenigstens einen Polyoxyethylen-glycerin-fettsäureester enthält. Als Feuchthaltemittel kommen beispielsweise Alkohole, Glycerin oder Glykole in Frage. Es hat sich gezeigt, dass eine Kombination der genannten Stoffe eine Flüssigkeit ergibt, die schnell abtrocknet und dabei in die Haut einzieht ohne diese auszutrocknen. Sie lässt sich problemlos mit einer kapillaren Spitze, etwa einer Faserpitze eines Auftragsgeräts auf die Haut aufbringen. Vorzugsweise wird ein Polyoxyethylen-glycerin-stearinsäureester verwendet.

Die beanspruchte Flüssigkeit zur Lippenpflege oder Lippenfärbung hat daher folgende Zusammensetzung (Gew.%):

| | |
|---|---|
| Feuchthaltemittel | 2 bis 40% |
| Polyoxyethylen-glycerin-fettsäureester | 0,1 bis 10% |
| Polyether-modifiziertes Polysiloxan | 0,2 bis 3% |
| Additive | 0 bis 22% |
| Wasser | ad 100% |

und einer Viskosität von max. 20 mPa.s (Brookfield, 20°C).

Eine Verbesserung im Hinblick auf die Permanenz, den Glanz und die Verträglichkeit der Flüssigkeit mit lipophilen Komponenten wird durch den Zusatz von polyether-modifiertem Polysiloxan erreicht. Bei besonders bevorzugten Zusammensetzungen sind 10 bis 30 Gew.% Feuchthaltemittel, 4 bis 10 Gew.% Polyoxyethylen-glycerin-fettsäureester und gegebenenfalls 0,5 bis 3 Gew.% polyether-modifiziertes Polysiloxan enthalten. Bei allen Zusammensetzungen wird eine Viskosität eingestellt, die maximal 20 mPa x s (Brookfield, T = 20°C) beträgt. Eine solche Flüssigkeit lässt sich problemlos in kapillaren Auftragsgeräten, also solchen, die nach dem Prinzip von Faserschreibern arbeiten, einsetzen. Als Farbmittel werden bei allen Formulierungen vorzugsweise für den Kosmetikbereich zugelassene Farbstoffe aus der Gruppe der "Säure-Farbstoffe" verwendet.

### Beispiel 1 (farblose Pflegeflüssigkeit):

| | |
|---|---|
| Methyl-4-Hydroxybenzoat | 0,20 Gew% |
| Propylenglykol | 15,00 Gew% |
| Polyoxyethylen-glycerin-stearinsäureester (CAS 68553-11-7) | 8,00 Gew% |
| Wasser | 74,48 Gew% |
| Polyether-modifiziertes Polysiloxan | 1,50 Gew% |
| Aloe Vera (Pulver) | 0,70 Gew% |
| Tocopherol | 0,10 Gew% |
| Zitronensäure | 0,02 Gew% |

Zur Herstellung werden die genannten Bestandteile beispielsweise in der angegebenen Reihenfolge zusammengemischt. Methyl-4-hydroxybenzoat dient als Konservierungsmittel. Als pflegende Zusätze sind Tocopherol, Zitronensäure als Synergist (für Tocopherol) und Aloe Vera enthalten. Der Polyoxyethylen-glycerin-stearinsäureester, der kürzer auch als PEG-30-Glyceriylstearat bezeichnet werden kann, ist beispielsweise von der Firma Goldschmidt unter dem Handelsnamen Tagat S erhältlich. Von dem gleichen Hersteller ist das polyethermodifizierte Polysiloxan unter dem Handelsnam Abil B 8851 erhältlich.

Bei den Beispielen 2 bis 4 handelt es sich nicht um Ausführüngsformen der Erfindung sondern um Formulierungen, die das Verständnis der Erfindung erleichtern.

### Beispiel 2 (violette Lippenfärbeflüssigkeit mit Aroma):

| | |
|---|---|
| Methyl-4-hydroxybenzoat | 0,50 Gew% |
| Ethanol | 20,00 Gew% |
| Polyoxyethylen-glycerin-stearinsäureester (CAS 68553-11-7) | 6,00 Gew% |
| Glycerin | 10,00 Gew% |
| Wasser | 59,62 Gew% |
| Polyether-modifiziertes Polysiloxan | 1,00 Gew% |
| Rum Aroma | 2,00 Gew% |
| Rotfarbstoff (CI 45410) | 0,50 Gew% |
| Orangefarbstoff (CI 15985) | 0,32 Gew% |
| Blaufarbstoff (CI 42090) | 0,06 Gew% |

### Beispiel 3 (braune Lippenfärbeflüssigkeit):

| | |
|---|---|
| Methyl-4-hydroxybenzoat | 0,50 Gew% |
| Ethanol | 20,00 Gew% |
| Polyoxyethylen-glycerin-stearinsäureester (CAS 68553-11-7) | 6,00 Gew% |
| Glycerin | 10,00 Gew% |
| Wasser | 59,40 Gew% |
| polyether-modifiziertes Polysiloxan | 1,00 Gew% |
| Aloe Vera (Pulver) | 0,50 Gew% |
| Rotfarbstoff (CI 45380) | 1,14 Gew% |
| Orangefarbstoff (CI 15985) | 1,26 Gew% |
| Blaufarbstoff (CI 42090) | 0,20 Gew% |

### Beispiel 4 (rote Lippenfärbeflüssigkeit)

| | |
|---|---|
| Methyl-4-hydroxybenzoat | 0,50 Gew% |
| Ethanol | 20,00 Gew% |
| Polyoxyethylen-glycerin-stearinsäureester (CAS 68553-11-7) | 5,50 Gew% |
| Glycerin | 10,00 Gew% |
| Wasser | 61,98 Gew% |
| Rotfarbstoff (CI 45380) | 0,90 Gew% |
| Orangefarbstoff (CI 15985) | 1,12 Gew% |

## Patentansprüche

1. Flüssigkeit zur Lippenpflege oder Lippenfärbung mit folgender Zusammensetzung (Gew.%):
| | |
|---|---|
| Feuchthaltemittel | 2 bis 40% |
| Polyoxyethylen-glycerin-fettsäureester | 0,1 bis 10% |
| polyether-modifiziertes Polysiloxan | 0,2 bis 3% |
| Additive | 0 bis 22% |
| Wasser | ad 100% |
und einer Viskosität von max. 20 mPa s (Brookfield, 20°C).

2. Flüssigkeit nach Anspruch 1,
**dadurch gekennzeichnet,**
**dass** Polyoxyethylen-glycerin-stearinsäureester enthalten ist.

3. Flüssigkeit nach Anspruch 1 oder 2,
**gekennzeichnet durch**
folgende Additive (Gew.%):
| | |
|---|---|
| Aromastoffe | 0 bis 5%. |
| Hautpflegemittel | 0 bis 5% |
| Farbmittel | 0 bis 10%. |
| Konservierungsmittel | 0 bis 2% |

4. Flüssigkeit nach einem der Ansprüche 1 bis 3, mit folgender Zusammensetzung (Gew.%) :
| | |
|---|---|
| Feuchthaltemittel | 10,0 bis 35,0% |
| Polyoxyethylen-glycerin-fettsäureester | 4,0 bis 10,0% |
| polyether-modifiziertes Polysiloxan | 0,5 bis 3,0% |
| Hautpflegemittel | 0 bis 1,0% |
| Farbmittel | 0 bis 5,0% |
| Aromastoff | 0 bis 4,0% |
| Konservierungsstoff | 0 bis 1,0% |
| Wasser | ad. 100% |

## Revendications

1. Liquide de soin ou de maquillage des lèvres ayant la composition suivante (en % en poids) :
| | |
|---|---|
| agent de conservation de l'humidité | 2 à 40 % |
| ester de glycérine et d'acide gras polyoxyéthyléné | 0,1 à 10 % |
| polysiloxane modifié par du polyétheroxyde | 0,2 à 3 % |
| additif | 0 à 22 % |
| eau | pour faire 100 % |
et une viscosité de 20mPa s au maximum (Brookfield, 20° C).

2. Liquide suivant la revendication 1,
**caractérisé en ce qu'**il contient de l'ester d'acide stéarique et de glycérine polyoxyéthyléné.

3. Liquide suivant la revendication 1 ou 2,
**caractérisé par**
des additifs suivants (en % en poids)
| | |
|---|---|
| arôme | 0 à 5 % |
| agent de soin principal | 0 à 5 % |
| colorant | 0 à 10 % |
| conservateur | 0 à 2 % |

4. Liquide suivant l'une des revendications 1 à 3,
ayant la composition suivante (en % en poids) :
| | |
|---|---|
| agent de conservation de l'humidité | 10,0 à 35,0 % |
| ester d'acide gras et de glycérine polyoxyéthyléné | 4,0 à 10, 0 % |
| polysiloxane modifié par du polyétheroxyde | 0,5 à 3,0 % |
| agent de soin principal | 0 à 1,0 % |
| colorant | 0 à 5,0 % |
| arôme | 0 à 4,0 % |
| conservateur | 0 à 1,0 % |
| eau | pour faire 100 % |

## Claims

1. Liquid for lip-care or lip colouring, having the following composition (% by weight):
| | |
|---|---|
| moisture retainer | 2 to 40% |
| polyoxyethylene glycerin fatty | |
| acid ester | 0.1 to 10% |
| polyether-modified polysiloxane | 0.2 to 3% |
| additives | 0 to 22% |
| water | ad 100% |
and a viscosity of max. 20 mPa s (Brookfield, 20°C).

2. Liquid according to Claim 1, **characterized in that** polyoxyethylene glycerin stearic acid ester is included.

3. Liquid according to Claim 1 or 2, **characterized by** the following additives (% by weight):
| | |
|---|---|
| aroma chemicals | 0 to 5% |
| skin-care agents | 0 to 5% |
| colorants | 0 to 10% |
| preservatives | 0 to 2% |

4. Liquid according to any one of Claims 1 to 3, having the following composition (% by weight):
| | |
|---|---|
| moisture retainer | 10.0 to 35.0% |
| polyoxyethylene glycerin fatty acid ester | 4.0 to 10.0% |
| polyether-modified polysiloxane | 0.5 to 3.0% |
| skin-care agent | 0 to 1.0% |
| colorant | 0 to 5.0% |
| aroma chemical | 0 to 4.0% |
| preservative | 0 to 1.0% |
| water | ad 100% |
